# EUROPEAN PATENT SPECIFICATION

(11) **EP 2 564 758 B1**
(45) Date of publication and mention of the grant of the patent: **25.06.2014**
(21) Application number: 12182019.5
(22) Date of filing: 28.08.2012
(51) Int. Cl.: A61B 1/00

(54) **Forceps plug for endoscope**
Zangenstecker für ein Endoskop
Embout de pinces pour endoscope

(30) Priority: 29.08.2011 JP 2011185583
(43) Date of publication of application: 06.03.2013
(73) Proprietor: Fujifilm Corporation, Minato-ku Tokyo 106-8620 (JP)
(72) Inventor: Yamane, Kenji, Kanagawa 258-8538 (JP)
(74) Representative: Klunker . Schmitt-Nilson . Hirsch

(56) References cited:
- JP-A- 2009 028 197
- JP-A- 2009 268 777
- JP-U- H0 239 701
- JP-U- H0 595 501
- US-A1- 2007 282 168

## Description

### BACKGROUND OF THE PRESENT INVENTION

### 1. Field of the present invention

The present invention relates to a forceps plug for an endoscope that is mounted on a metal opening provided at an inlet of a treatment tool-insertion channel and is used when a treatment tool is inserted into a treatment tool-insertion channel of an endoscope.

### 2. Description of the Related Art

In the past, a medical endoscope has been adapted not only to be capable of observing a condition in the body but also to be capable of collecting a tissue cell or performing a predetermined treatment when a lesioned part or the like is present in the body. For example, for the insertion of a treatment tool into the body under the control of the endoscope, a treatment tool-insertion channel into which a forceps, other treatment tools, and the like are inserted is formed in an insertion unit of the endoscope. A base end portion of the treatment tool-insertion channel communicates with a treatment tool introducing portion that is formed at a main body operating unit, and a tip thereof and observation portions and the like, which are formed on the apical surface or tip side surface of the insertion unit, are opened as a treatment tool outlet.

As a technique of the forceps plug in the related art, there is a technique disclosed in, for example, JP2009-28197A. The structure of a forceps plug where a main body portion in which a closing film including a slit is formed and a cap portion in which a closing film including an insertion hole is formed are provided and the main body portion and the cap portion are connected to each other by a deformable connection string-like member is proposed in JP2009-28197A. In this invention, both the smoothness of insertion of a treatment tool and the complete prevention of the scattering or the like of contaminated liquid, which occurs due to the sudden rise of the pressure (so-called water hammer phenomenon) caused by the patient's belching and the like in the treatment tool-insertion channel, (hereinafter, abbreviated as the scattering or the like of contaminated liquid), are achieved in the case of a treatment tool having a certain diameter.

As a technique that solves the above-mentioned problem, there is a technique disclosed in, for example, JP1998-192229A (JP-H10-192229A). The structure of a forceps plug where a closing film including two or more insertion holes or slits is formed in a main body portion is proposed in JP1998-192229A (JP-H10-192229A). In this invention, the complete prevention of the scattering or the like of contaminated liquid is achieved in the cases of various treatment tools.

JP 2 009 268 777 A discloses a forceps plug comprising a main body portion and two cap portions.

### SUMMARY OF THE PRESENT INVENTION

A forceps plug of an endoscope that is effective in the cases of various treatment tools is required to meet a request such as the diversification or complication of an operation using an endoscope. However, the technique in the related art has the following problems.

When the forceps plug disclosed in JP2009-28197A is used, there is a problem in that both the smoothness of insertion of a treatment tool and the complete prevention of the scattering or the like of contaminated liquid are insufficient in the case of treatment tools having diameters except for a certain diameter when a plurality of treatment tools need to be used to perform a series of operations.

When the forceps plug disclosed in JP1998-192229A (JP-H10-192229A) is used, there is a problem in that the forceps plug should be designed to have a particularly large size since the size of the closing film needs to be increased in design in order to form a plurality of treatment tool insertion portions at the closing film formed in the main body portion; there is a problem in that the smoothness of insertion of a treatment tool is insufficient since the metal opening and the insertion hole of the forceps plug are not present coaxially; and there is a problem in that there is a possibility that fragments of the closing film falls into the body of a patient since the closing film is easily broken as the plurality of treatment tool insertion portions are formed at the closing film.

There is a demand for the achievement of both the smoothness of insertion of a treatment tool and the complete prevention of the scattering or the like of contaminated liquid in the cases of various treatment tools, without particularly increasing the size of a forceps plug in design and without causing a new problem that a forceps plug is easily broken.

In order to solve the above-mentioned problems, according to an aspect of the present invention, there is provided a forceps plug for an endoscope that is mounted on a metal opening communicating with an insertion channel provided in an endoscope for the insertion of a treatment tool when being used. The forceps plug for an endoscope includes a main body portion and a plurality of cap portions. The main body portion includes a mounting portion that is to be mounted on the metal opening and formed at one end thereof and a cap mounting portion that protrudes in a cylindrical shape and is formed at the other end thereof. A closing film having a slit normally closed is formed between the mounting portion and the cap mounting portion, and the slit is pushed and opened by the insertion of the treatment tool so that the treatment tool passes through the slit. The plurality of cap portions are connected to the main body portion by flexible strip-like portions, respectively and are formed of insertion holes for inserting the treatment tool with different inner diameters one by one at the centers of the cap portions, where said cap portions extend continuously around the respective insertion hole and are attachable and detachable on the cap mounting portion in an alternative manner.

When the forceps plug according to the aspect of the present invention is used, the main body portion, which includes a closing film including a slit normally closed, is mounted on the metal opening of an endoscope and a cap portion in which an insertion hole having an inner diameter optimal for a treatment tool is formed is selected and mounted on the main body portion. Here, the insertion hole having the optimal inner diameter means an insertion hole having an inner diameter optimal for the achievement of both the smoothness of insertion of a treatment tool and the complete prevention of the scattering or the like of contaminated liquid. Further, since the forceps plug according to the aspect of the present invention is divided into the main body portion and the cap portions, the main body portion does not need to be mounted or detached and the cap portion can be appropriately changed into a cap portion in which an insertion hole having an optimal inner diameter is formed according to the size of a treatment tool when a treatment tool is changed.

When the metal opening of the endoscope is divided into a plurality of sections, a forceps plug in which main body portions of which the number is equal to the number of the plurality of sections are formed and which includes cap portions formed as described above can be used. Since the forceps plug according to the aspect of the present invention is divided into the main body portion and the cap portions, the main body portion does not need to be mounted or detached and the cap portion can be appropriately changed into a cap portion in which an insertion hole having an optimal inner diameter is formed according to the size of a treatment tool when a treatment tool is changed.

Further, since a mark corresponding to an inner diameter of an insertion hole, which is formed in the cap portion corresponding to each of the strip-like portions, is made, a cap portion optimal for a treatment tool can be more accurately and more quickly selected and mounted on the main body portion. Here, the cap portion corresponding to each of the strip-like portions means each cap portion connected to each strip-like portion.

Furthermore, since the respective cap portions are colored in different colors, a cap portion optimal for a treatment tool can be more accurately and more quickly selected and mounted on the main body portion.

According to the aspect of the present invention, each of the cap portions, which include insertion holes which are formed one by one at the centers thereof and have inner diameters different from each other and into which the treatment tool is inserted, is connected to the main body portion by the flexible strip-like portion. Accordingly, in the cases of various treatment tools, the main body portion does not need to be mounted on or detached from the metal opening of the endoscope and both the smoothness of insertion of a treatment tool and the complete prevention of the scattering or the like of contaminated liquid can be achieved.

Further, since a slit normally closed is formed at the closing film of the main body portion, a closing film of the main body portion including a slit into which the forceps is not inserted prevents liquid from leaking when the kind of a cap is changed in a case.

According to another aspect of the present invention, since a mark corresponding to an inner diameter of an insertion hole, which is formed in the cap portion corresponding to each of the strip-like portions, is made, a cap portion optimal for a treatment tool can be more accurately and more quickly selected and mounted on the main body portion. Accordingly, in the cases of various treatment tools, the main body portion does not need to be mounted on or detached from the metal opening of the endoscope and both the smoothness of insertion of a treatment tool and the complete prevention of the scattering or the like of contaminated liquid can be further achieved.

Furthermore, according to another aspect of the present invention, since the respective cap portions are colored in different colors, a cap portion optimal for a treatment tool can be more accurately and more quickly selected and mounted on the main body portion. Accordingly, in the cases of various treatment tools, the main body portion does not need to be mounted on or detached from the metal opening of the endoscope and both the smoothness of insertion of a treatment tool and the complete prevention of the scattering or the like of contaminated liquid can be further achieved.

### BRIEF DESCRIPTION OF THE DRAWINGS

Fig. 1 is a schematic view of an endoscope system according to the present invention.
Fig. 2 is a perspective view of a forceps plug according to a first embodiment of the present invention.
Fig. 3 is a cross-sectional view of the forceps plug according to the first embodiment of the present invention.
Fig. 4 is a cross-sectional view of the forceps plug into which a forceps according to the first embodiment of the present invention has been inserted.
Fig. 5 is a perspective view of a forceps plug according to a second embodiment of the present invention.
Fig. 6 is a perspective view of a forceps plug according to a third embodiment of the present invention.
Fig. 7 is a perspective view of a forceps plug according to a fourth embodiment of the present invention.
Fig. 8 is a perspective view of a forceps plug according to a fifth embodiment of the present invention.
Fig. 9 is a perspective view of a forceps plug according to the fifth embodiment of the present invention.

### DESCRIPTION OF THE PREFERRED EMBODIMENTS

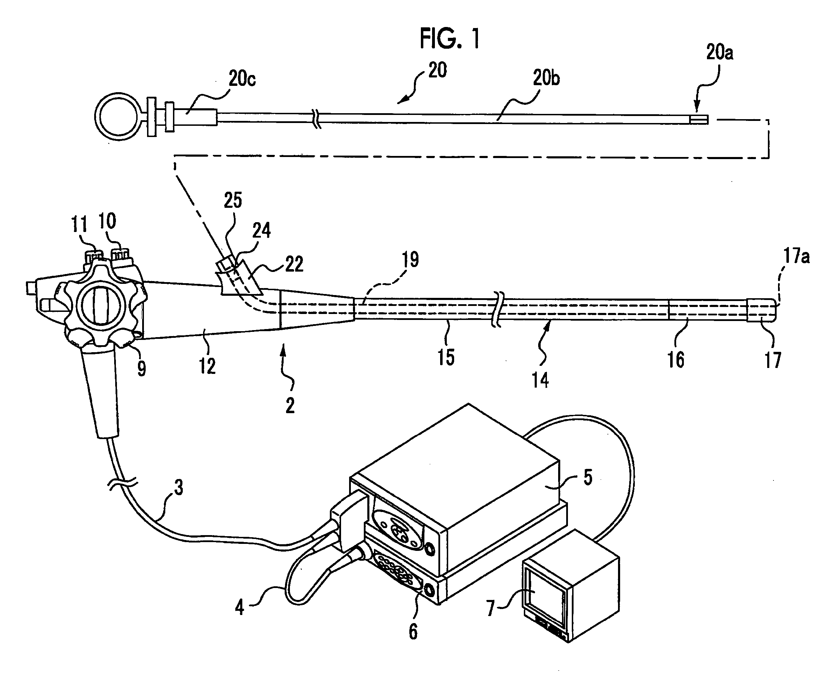

As shown in Fig. 1, an endoscope 2 is connected to a light source device 5 and an endoscope processor 6 by a universal cord 3, a cable 4 and the like when being used. An operator can observe an image, which is acquired by the endoscope 2, on a monitor 7 for displaying an image. The endoscope 2 includes a hand operation unit 12 that is provided with an angle knob 9, an air supply/water supply button 10, a suction button 11, and the like; and an insertion unit 14 that is connected to the hand operation unit 12 and inserted into a body cavity. The insertion unit 14 includes a soft section 15, a bending section 16, and a tip hard section 17 in this order from the base end thereof close to the hand operation unit 12. The bending section 16 is bent so as to interlock with the operation of the angle knob 9. An optical system, an imaging device including an imaging element, and the like are built in the tip hard section 17.

The endoscope 2 is provided with an insertion channel 19 for the insertion of a treatment tool so that the operator can perform an appropriate treatment for an affected part while observing the image of the affected part on the monitor 7. Various treatment tools, which are previously prepared for an endoscope, such as a high-frequency scalpel and a high-frequency snare, other than a biopsy forceps can be inserted into the insertion channel 19 when being used. For example, when a forceps 20 is selected according to a treatment for an affected part among these treatment tools, the forceps 20 is inserted into the insertion channel 19 through a forceps port 22 formed at the hand operation unit 12.

The forceps 20 includes a cup 20a for biopsy collection, a flexible insertion portion 20b, and an operation portion 20c in which an operation handle and a link mechanism are built, in this order from the tip thereof. The cup 20a protrudes from an outlet 17a, which is formed at the apical surface of the tip hard section 17, and the operation portion 20c is exposed to the outside from the forceps port 22. The operator can perform an appropriate treatment by operating the operation portion 20c while observing the affected part on the monitor 7 and confirming the operation of the cup 20a.

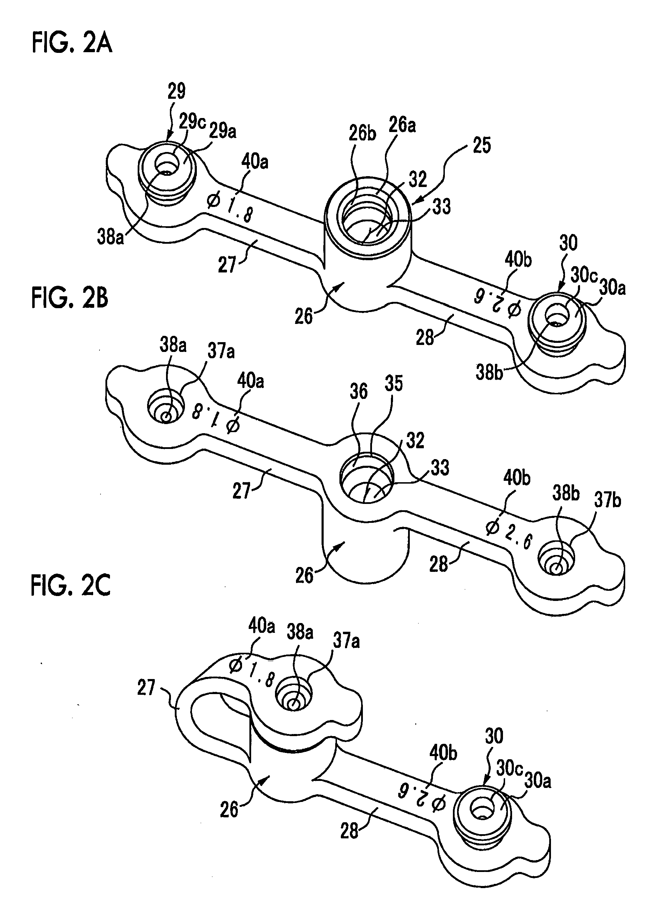

A metal opening 24, which protrudes in a cylindrical shape, is formed on the outer portion of the forceps port 22 so that water, air, or body fluid, which flows into the insertion channel 19 through the outlet 17a and flows backward, is not ejected from the forceps port 22 when the forceps 20 is used. A forceps plug 25 shown in Fig. 2 is mounted on the metal opening 24. The forceps plug 25 is made of a material having high flexibility and elasticity, such as rubber or elastomer, and is mounted in close contact with the metal opening 24 with no gap so that fluid does not leak out from a mounting portion between the forceps plug 25 and the metal opening 24.

In the first embodiment, as shown in Fig. 2A, the forceps plug 25 includes a main body portion 26 that is formed in a cylindrical shape as a whole, and first and second cap portions 29 and 30 that are connected to both sides of the main body portion 26 by strip-like portions 27 and 28. The first and second cap portions 29 and 30 include cylindrical insertion portions 29a and 30a that are formed in the same shape so as to protrude upward from the strip-like portions 27 and 28, and exposed openings 29c and 30c also have the same inner diameter. Meanwhile, all of the main body portion 26, the strip-like portions 27 and 28, and the first and second cap portions 29 and 30 are molded integrally in the embodiment shown in Fig. 2. However, an integrated molded article of the strip-like portion 27 and the first cap portion 29 and an integrally molded article of the strip-like portion 28 and the second cap portion 30 may be connected to an integrally molded article of the main body portion 26.

An insertion portion 29a or 30a of any one of the first and second cap portions 29 and 30 is selected and inserted into an opening 26a of the main body portion 26 as shown in Fig. 2C, and a holding portion 26b, which is elastically engaged with and holds the inserted insertion portion 29a or 30a, is formed in the opening 26a. As a result, the opening 26a of the main body portion 26 and the holding portion 26b formed in the opening 26a function as a cap mounting portion for the first and second cap portions 29 and 30. A closing film 33, which includes a slit 32 normally closed at the center thereof, is further formed in the main body portion 26. Since the slit 32 is urged in a direction where the slit 32 is closed, the closing film 33 is opened when being pushed and opened by an external force.

The bottom side of the forceps plug 25 is shown in Fig. 2B, and an opening 35 into which the metal opening 24 is inserted is exposed to the bottom of the main body portion 26. An engaging portion 36, which is elastically brought into close contact with and is engaged with a step portion of the metal opening 24, is formed in the opening 35, and the opening 35 and the engaging portion 36 form a mounting portion that mounts the forceps plug 25 on the metal opening 24. Meanwhile, the above-mentioned closing film 33 with the slit 32 is also observed from the bottom side through the opening 35. Further, openings 37a and 37b into which the forceps 20 is inserted from the tip thereof are exposed to the bottom sides of the first and second cap portions 29 and 30. The inner diameters of these openings 37a and 37b are the same as each other, and are larger than the outer diameters of various treatment tools that are inserted into the insertion channel 19 and can be used together with the endoscope 2.

The openings of insertion holes 38a and 38b having different inner diameters are exposed in the openings 37a and 37b. The inner diameters of the insertion holes 38a and 38b are determined according to the outer diameters of the insertion portion 20b of the treatment tool that can be suitably used together with the forceps plug 25; and marks 40a and 40b showing the outer diameter of the insertion portion of the forceps 20, which can be used, are made on the front and back of each of the strip-like portions 27 and 28, as marks corresponding to the inner diameter of the insertion hole. Accordingly, when the outer diameter of the insertion portion of a forceps 20 to be used this time is 1.8 mm, an operator may select and mount the cap portion 29 on the main body portion 26 as shown in Fig. 2C after confirming the mark 40a. Meanwhile, not only the numerical value of the external dimension of the insertion portion of a forceps to be used but also the inner diameter and the like of the insertion hole may be used as a mark corresponding to the inner diameter of the insertion hole; and an object to be marked may be a number, a bar code, a symbol, a letter, a figure, an emblem, or the combination thereof, other than a numerical value such as an external dimension. Further, to reduce the length of the strip-like portion 27, it is advantageous to mount the first cap portion 29 on the main body portion 26 while reversing and bending the strip-like portion 27 as shown in Fig. 2C without wrenching the strip-like portion 27.

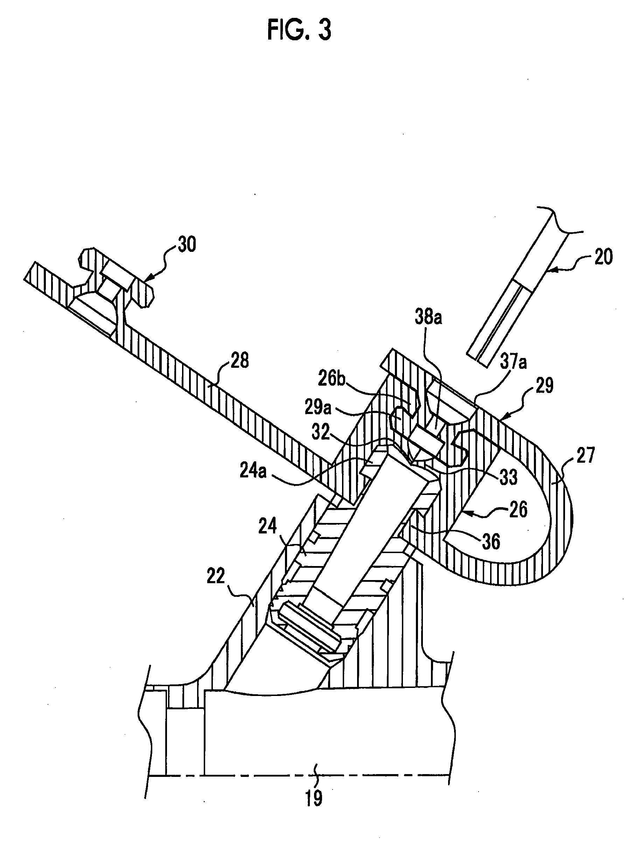

As shown in Fig. 3, the forceps plug 25 is mounted on the metal opening 24 that is firmly fixed to the forceps port 22. A flange portion 24a is formed at the upper end of the metal opening 24, and the engaging portion 36 formed at the main body portion 26 of the forceps plug 25 is fitted to a step portion that is formed below the flange portion 24a. Accordingly, the metal opening 24 comes into close contact with and is connected to the forceps plug 25 so as to be wrapped by the main body portion 26. The first cap portion 29, which is selected according to the forceps 20, is mounted on the cap mounting portion that is exposed to the upper portion of the main body portion 26. When the first cap portion 29 is mounted on the cap mounting portion, the insertion portion 29a may be pushed from the opening 26a of the main body portion 26. As shown in Fig. 3, the holding portion 26b formed at the main body portion 26 is elastically engaged with the step portion formed at the insertion portion 29a, so that the first cap portion 29 is fixed to the main body portion 26 so as to seal the main body portion 26.

After the first cap portion 29 is mounted on the main body portion 26, the forceps 20 is inserted into the insertion channel 19 from the opening 37a of the first cap portion 29. There is a case where the diameter of the cup 20a formed at the tip of the forceps 20 is larger than that of the insertion portion 20b. However, since the cup 20a is short, the forceps 20 can be relatively easily inserted into the insertion hole 38a. Moreover, when the forceps 20 is pushed, the cup 20a pushes and opens the slit 32 of the closing film 33, passes through the main body portion 26 and the metal opening 24, and reaches the insertion channel 19. After that, the forceps 20 can be easily inserted into the insertion channel 19 by the guiding operation of the inner wall of the insertion channel 19.

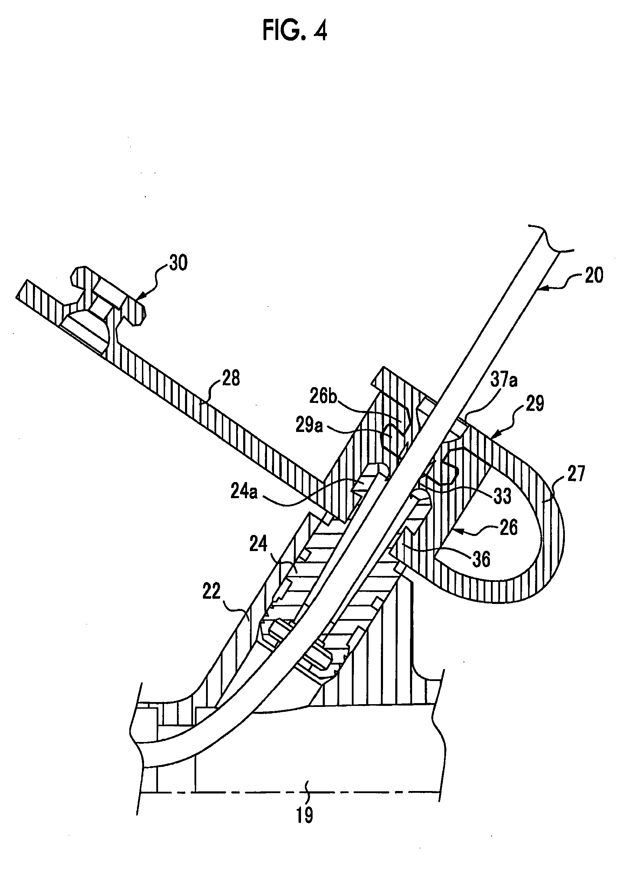

After the forceps 20 is inserted by a prescribed length, the insertion portion 20b of the forceps 20 is inserted into the insertion hole 38a of the cap portion 29 as shown in Fig. 4. The inner diameter of the insertion hole 38a is determined according to the outer diameter of the insertion portion 20b of the forceps 20 so that the insertion portion 20b is easily inserted and a gap is almost not formed between the insertion portion 20 and the insertion hole 38a. Accordingly, almost no water, body fluid, fine foreign materials, or the like, which have flowed backward from the insertion channel 19, are ejected from the forceps port 22 while the forceps 20 is used, when the forceps 20 is pulled from the insertion channel 19 after the use of the forceps 20, or the like.

Further, since the closing film 33 is formed in the main body portion 26, the slit 32 comes into contact with the outer peripheral surface of the insertion portion 20b while being bent by an elastic force. Accordingly, a gap is sufficiently small even in this portion. For this reason, since there is less concern that water or body fluid having flowed backward is directly ejected to the gap between the insertion hole 38a and the insertion portion 20b, a possibility that surroundings are contaminated during a treatment or an aftertreatment can be sufficiently reduced. Of course, when the outer diameter of the insertion portion of a treatment tool is 2.6 mm, the second cap portion 30 including the insertion hole 38b having an inner diameter suitable for the outer diameter may be mounted on the main body portion 26. Even in this case, the closing film 33 acts in completely the same manner and approximately the same advantages can be obtained.

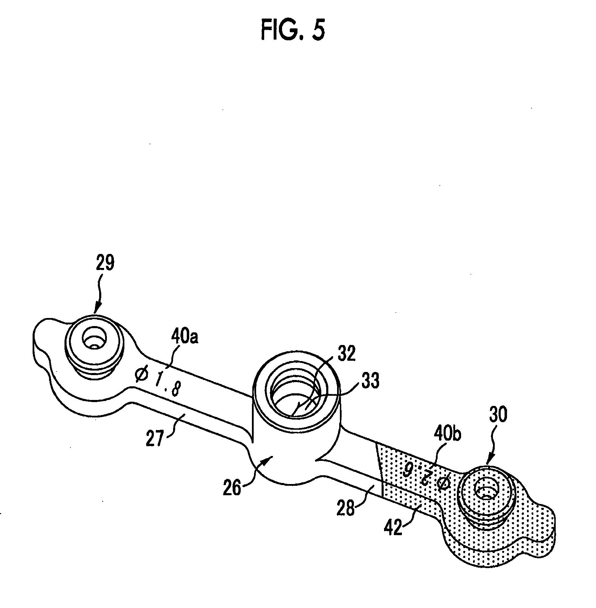

In a second embodiment, a part of the above-mentioned first embodiment is colored as shown in Fig. 5. Accordingly, the shape and dimensions are completely common to the first and second embodiments and the portions common to the first and second embodiments are denoted by the same reference numerals. In this embodiment, a colored portion 42 is formed at one, which has a larger inner diameter between the inner diameters of the insertion holes 38a and 38b, of the first and second cap portions 29 and 30. The colored portion 42 may be formed by two-color molding at the time of the molding of the forceps plug other than general painting. If attention is paid in this way, it is effective in preventing misuse as compared to a case where only the marks 40a and 40b of dimensions are made merely.

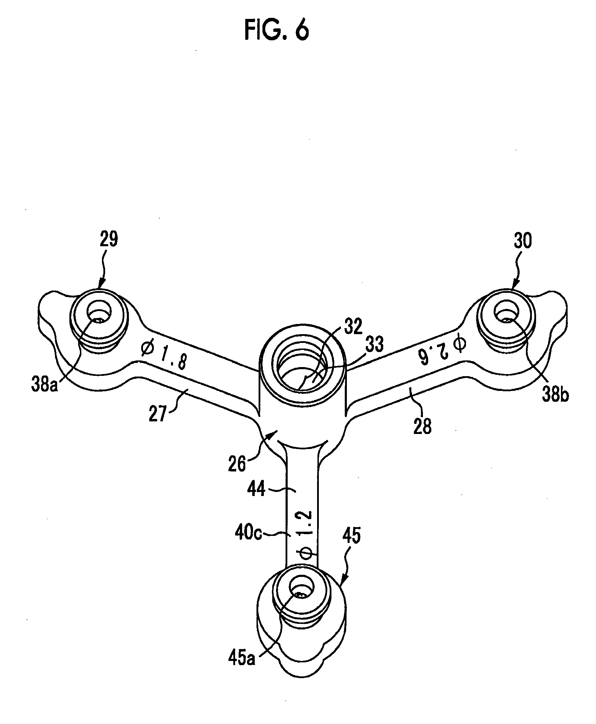

In a third embodiment, three kinds of cap portions are integrated with a main body portion 26 at an interval of 120° as shown in Fig. 6, and a strip-like portion 44 and a third cap portion 45 are formed at the main body portion 26 in addition to strip-like portions 27 and 28 and first and second cap portions 29 and 30 that are completely the same as those of the embodiment shown in Fig. 2. The basic configuration of the third cap portion 45 is common to the first and second cap portions 29 and 30, and the inner diameter of an insertion hole 45a into which a treatment tool is to be inserted is set to a size optimal for the outer diameter of an insertion portion of a treatment tool having an outer diameter of 1.2 mm. Further, a mark 40c thereof is also made on the strip-like portion 44. Accordingly, according to this forceps plug, even when any one of three kinds of treatment tools of which the outer diameters of the insertion portions are 1.2 mm, 1.8 mm, and 2.6 mm is used, like in the previous embodiment, unprepared contamination can be prevented during a treatment or at the time of an aftertreatment by the use of an optimal cap portion according to the outer diameter of the insertion portion of the treatment tool to be used. Meanwhile, a fourth cap portion including an insertion hole having another inner diameter or other cap portions may be further connected to the main body portion 26 together with strip-like portions so as to be capable of coping with the four or more kinds of treatment tools.

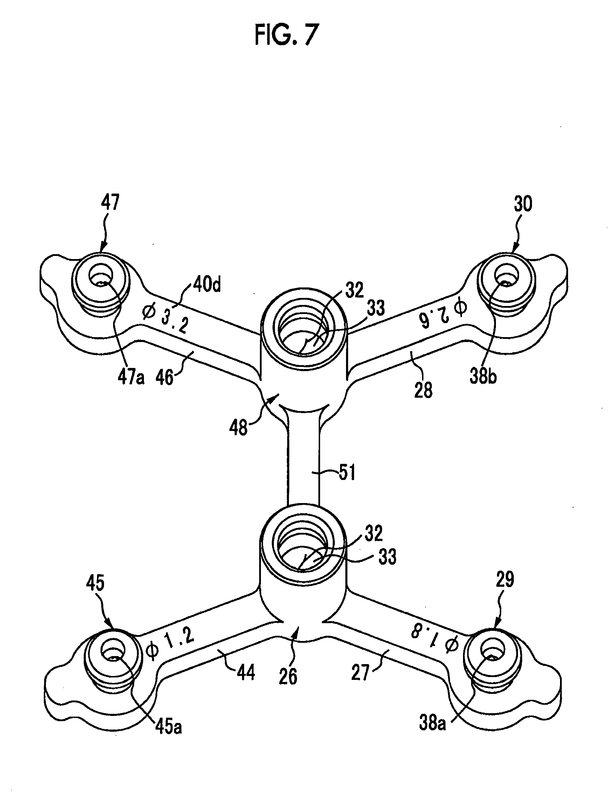

In a fourth embodiment, a main body portion 48 is integrally connected to a main body portion 26 by a strip-like portion 51 as shown in Fig. 7. All shape and size are common to the main body portions 26 and 48, and two kinds of cap portions are connected to each of the main body portions 26 and 48. First cap portions 29, 30, and 45, which are connected by strip-like portions 27, 28, and 44, are common to the forceps plug shown in Fig. 6. The main body portion 48 newly includes a strip-like portion 46 and a fourth cap portion 47, and a new mark 40d is made on the strip-like portion 46. The inner diameter of an insertion hole 47a of the fourth cap portion 47 is set to a dimension optimal for the outer diameter of an insertion portion of a treatment tool that is 3.2 mm. Accordingly, if the fourth cap portion 47 is used after the main body portion 48 is mounted on the metal opening 24, a treatment tool of which the outer diameter of the insertion portion is 3.2 mm can also be used.

Moreover, in an endoscope where an insertion channel 19 is branched from the middle thereof or branched from a portion of the forceps port 22 and two metal openings 24 protrude, the main body portions 26 and 48 of this forceps plug can be mounted on the respective metal openings 24. Further, appropriate cap portions are mounted on the respective cap insertion portions, so that two kinds of treatments can be effectively used with the two metal openings 24 at the same time.

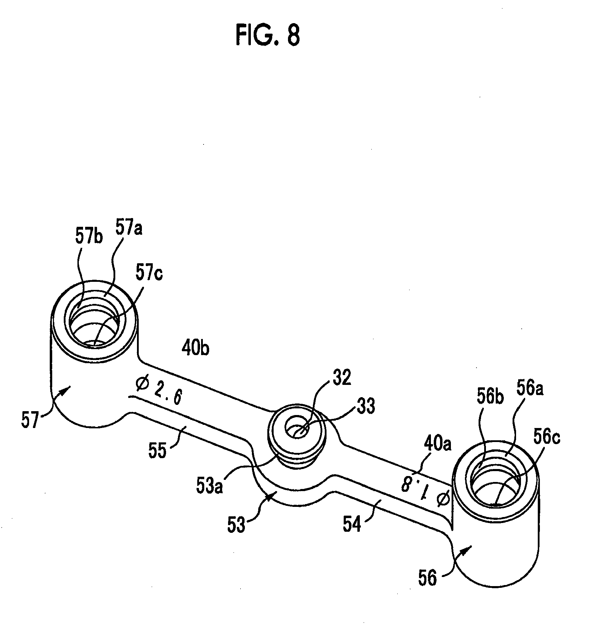

In a fifth embodiment, as shown in Fig. 8, a cap mounting portion 53a protrudes from the upper surface of a main body portion 53 and cap portions 56 and 57 can be mounted on the cap mounting portion 53a so as to cover the cap mounting portion 53a. A closing film 33, which includes a slit 32 and is always closed, is formed in the main body portion 53 like in the above-mentioned embodiments. The cap portions 56 and 57 are integrally connected to the main body portion 53 by strip-like portions 54 and 55. Holding portions 56b and 57b, which are engaged with concave and convex portions of the cap mounting portion 53a of the main body portion 53, are formed in openings 56a and 57a of the cap portions 56 and 57. Further, insertion holes 56c and 57c, which have inner diameters suitable when the outer diameters of the insertion portions of treatment tools to be inserted are 1.8 mm and 2.6 mm, are formed in the respective cap portions 56 and 57.

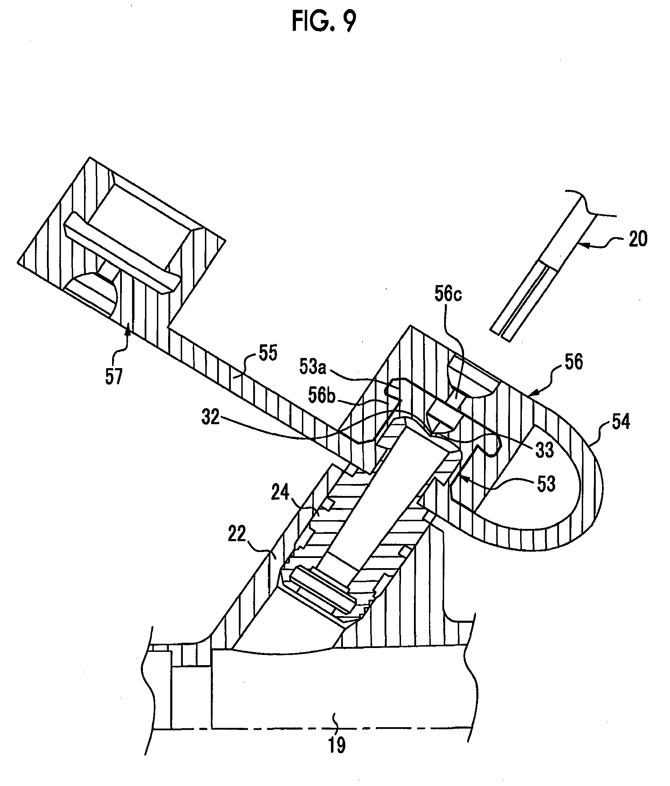

When the cap portion 56 is mounted on the main body portion 53 as shown in Fig. 9, a large-diameter cylindrical portion of the cap portion 56 is engaged with the cap mounting portion 53a so as to wrap the cap mounting portion 53a that protrudes upward from the main body portion 53 in the shape of a cylinder having a small diameter. Further, the insertion hole 56c of the cap portion 56 is positioned immediately above the slit 32 that is formed at the center of the closing film 33 of the main body portion 53. Accordingly, a forceps 20, of which the outer diameter of the insertion portion 20b is 1.8 mm, can be easily inserted into the insertion hole 56c. Furthermore, fluid, foreign materials, or the like, which have flowed backward from the insertion channel 19, do not leak out from a gap between the insertion hole 56c and the insertion portion 20b of the forceps 20 like in the above-mentioned embodiments.

Moreover, when the cap portion 56 is mounted on the cap mounting portion 53a of the main body portion 53, the cap mounting portion 53a is pressed from the outer peripheral side by the cap portion 56. Accordingly, an urging force is applied to the slit 32, which is pushed and opened by the insertion of the insertion portion 20b of the forceps 20, in a closing direction, so that the interruption performance of the closing film 33 is improved. Meanwhile, it goes without saying that completely the same function is obtained if a cap portion 57 is mounted on the main body portion 53 and used instead of the cap portion 56 when a treatment tool of which the outer diameter of an insertion portion is 2.6 mm is used.

The first to fifth embodiments have been described above. However, the present invention is not limited to these embodiments, and design based on the same technical idea may be made.

## Claims

1. A forceps plug for an endoscope that is mountable on a metal opening communicating with an insertion channel provided in an endoscope for the insertion of a treatment tool when being used, the forceps plug for an endoscope comprising:
a main body portion including a mounting portion that is configured to be mounted on the metal opening and formed at one end thereof and a cap mounting portion that protrudes in a cylindrical shape and is formed at the other end thereof, a closing film having a slit normally closed being formed between the mounting portion and the cap mounting portion, and the slit being configured to be pushed and opened by the insertion of the treatment tool so that the treatment tool passes through the slit; and **characterised in that**
a plurality of cap portions that are connected to the main body portion by flexible strip-like portions, respectively, and are formed of insertion holes with different inner diameters for inserting the treatment tool one by one at the centers of the cap portions, where said cap portions extend continuously around the respective insertion hole and are attachable and detachable on the cap mounting portion in an alternative manner.

2. The forceps plug for an endoscope according to claim 1,
wherein the main body portion, the strip-like portions, and the cap portions are made of an elastic material so as to be integrally molded.

3. The forceps plug for an endoscope according to claim 2,
wherein the cap portion is mounted on the cap mounting portion by reversing and bending the strip-like portion.

4. The forceps plug for an endoscope according to claims 1 to 3,
wherein a mark corresponding to an inner diameter of an insertion hole, which is formed in the cap portion corresponding to each of the strip-like portions, is present.

5. The forceps plug for an endoscope according to claims 1 to 4,
wherein the respective cap portions are colored in colors different from each other.

## Patentansprüche

1. Zangenstopfen für ein Endoskop, der an einer Metallöffnung anbringbar ist, die im Gebrauch mit einem Einführkanal in einem Endoskop zum Einführen eines Behandlungswerkzeugs kommuniziert, wobei der Zangenstopfen für ein Endoskop umfasst:
einen Hauptkörperteil mit einem Anbringabschnitt, der konfiguriert ist zur Anbringung an der Metallöffnung und an seinem einen Ende ausgebildet ist, und mit einem Deckelanbringabschnitt, der in zylindrischer Form absteht und an seinem anderen Ende ausgebildet ist, mit einem Verschlussfilm, der einen normalerweise geschlossenen Schlitz aufweist und zwischen dem Anbringabschnitt und dem Deckelanbringabschnitt ausgebildet ist, wobei der Schlitz konfiguriert ist, um durch das Einführen des Behandlungswerkzeugs stoßend geöffnet zu werden, so dass das Behandlungswerkzeug durch den Schlitz hindurchtritt; **dadurch gekennzeichnet, dass**
mehrere Deckelabschnitte, die mit dem Hauptkörperteil durch flexible, streifenähnliche Teile verbunden sind, und die aus Einführlöchern mit unterschiedlichen Innendurchmessern zum Einführen des Behandlungwerkzeugs eines nach dem anderen in den Mitten der Deckelabschnitte ausgebildet sind, wobei die Deckelabschnitte sich durchgängig um das jeweilige Einführloch herum erstrecken und an dem Deckelanbringabschnitt abwechselnd anbringbar und von diesem lösbar sind.

2. Zangenstopfen für ein Endoskop nach Anspruch 1, bei dem der Hauptkörperteil, die streifenähnlichen Teile und die Deckelabschnitte aus einem elastischen Werkstoff bestehen und einstückig geformt sind.

3. Zangenstopfen für ein Endoskop nach Anspruch 2, bei dem der Deckelabschnitt an dem Deckelanbringabschnitt durch Zurückbiegen des streifenähnlichen Teils angebracht ist.

4. Zangenstopfen für ein Endoskop nach Anspruch 1 bis 3, bei dem eine einem Innendurchmesser eines Einführlochs entsprechende Markierung vorhanden ist, die in dem Deckelabschnitt entsprechend jedem der streifenähnlichen Abschnitte ausgebildet ist.

5. Zangenstopfen für ein Endoskop nach Anspruch 1 bis 4, bei dem die jeweiligen Deckelabschnitte in voneinander verschiedenen Farben gefärbt sind.

## Revendications

1. Embout de pince pour endoscope pouvant être monté sur une ouverture métallique communiquant avec un canal d'insertion prévu dans un endoscope pour l'insertion d'un instrument de traitement lorsque celui-ci est utilisé, l'embout de pince pour endoscope comprenant :
une partie formant corps principal incluant une partie de montage configurée pour être montée sur l'ouverture métallique et formée sur une extrémité de celle-ci, ainsi qu'une partie de montage à capuchon qui fait saillie sous une forme cylindrique et est formée sur l'autre extrémité de celle-ci, un film de fermeture présentant une fente normalement fermée entre la partie de montage et la partie de montage à capuchon, et la fente étant configurée pour être poussée et ouverte par l'insertion de l'instrument de traitement, de sorte que l'instrument de traitement passe à travers la fente, et **caractérisé par**
une pluralité de parties à capuchon reliées à la partie formant corps principal respectivement par des parties flexibles similaires à des bandelettes et formées de trous d'insertion présentant des diamètres intérieurs différents, pour insérer l'instrument de traitement un à un au niveau des centres des parties à capuchon, où lesdites parties à capuchon s'étendent de manière continue autour du trou d'insertion respectif et peuvent être fixées sur la partie de montage à capuchon, et désolidarisées de celle-ci, d'une autre manière.

2. Embout de pince pour endoscope selon la revendication 1,
dans lequel la partie formant corps principal, les parties similaires à des bandelettes et les parties à capuchon sont fabriquées dans un matériau élastique de manière à être moulées d'un seul tenant.

3. Embout de pince pour endoscope selon la revendication 2,
dans lequel la partie à capuchon est montée sur la partie de montage à capuchon en retournant et recourbant la partie similaire à une bandelette.

4. Embout de pince pour endoscope selon l'une quelconque des revendications 1 à 3, dans lequel se trouve un repère correspondant à un diamètre intérieur d'un trou d'insertion, lequel est formé dans la partie à capuchon correspondant à chacune des parties similaires à des bandelettes.

5. Embout de pince pour endoscope selon l'une quelconque des revendications 1 à 4, dans lequel les parties à capuchon respectives sont colorées en des couleurs différentes les unes des autres.
